# EUROPEAN PATENT SPECIFICATION

(11) **EP 2 776 580 B1**
(45) Date of publication and mention of the grant of the patent: **04.05.2016**
(21) Application number: 12788630.7
(22) Date of filing: 06.11.2012
(51) Int. Cl.: C12Q 1/68

(54) **MICRO -RNAS AS MARKER FOR PLATELET ACTIVITY**
MIRNAS ALS MARKER DER THROMBOZYTENAKTIVITÄT
MICRO-ARNS COMME MARQUEURS DE L'ACTIVITÉ DES PLAQUETTES SANGUINES.

(30) Priority: 11.11.2011 GB 201119571; 13.07.2012 GB 201212539
(43) Date of publication of application: 17.09.2014
(73) Proprietor: Micro-Signature Ltd., Sutton Surrey SM1 4HX (GB)
(72) Inventor: MAYR, Manuel, London SE5 9NU (GB)
(74) Representative: Tombling, Adrian George
(86) International application number: PCT/GB2012/052754
(87) International publication number: WO 2013/068730

(56) References cited:
- WO-A2-2005/118806
- WO-A2-2007/109236
- WO-A2-2011/133036
- A. A. KONDKAR ET AL: "VAMP8/endobrevin is overexpressed in hyperreactive human platelets: suggested role for platelet microRNA", JOURNAL OF THROMBOSIS AND HAEMOSTASIS, vol. 8, no. 2, 1 February 2010 (2010-02-01), pages 369-378, XP055051397, ISSN: 1538-7933, DOI: 10.1111/j.1538-7836.2009.03700.x
- S. NAGALLA ET AL: "Platelet microRNA-mRNA coexpression profiles correlate with platelet reactivity", BLOOD, vol. 117, no. 19, 17 March 2011 (2011-03-17), pages 5189-5197, XP055051289, ISSN: 0006-4971, DOI: 10.1182/blood-2010-09-299719
- OSMAN ABDIMAJID; FALKER KNUT: "Characterization of human platelet microRNA by quantitative PCR coupled with an annotation network for predicted target genes", PLATELETS, vol. 22, no. 6, 1 September 2011 (2011-09-01), pages 433-441, XP009166607,
- TANRIVERDI KAHRAMAN; MORIN KRISTINE; BEAULIEU LEA M; FREEDMAN JANE E: "Platelet Activation Regulates Levels of MicroRNA", CIRCULATION, vol. 118, no. 18, 28 August 2008 (2008-08-28), page s407, XP009166631,
- TANRIVERDI KAHRAMAN; VITSERA OLGA; IAFRATI MARK D; REX SYBILLE; BLAIR PRICE S; FREEDMAN JANE E: "Platelet MicroRNA is altered by thrombin-induced aggregation", CIRCULATION, vol. 114, no. 18, 31 October 2006 (2006-10-31), pages 27-28, XP009166639,

## Description

The present invention relates to a method of determining platelet activity. The present invention also relates to a method of monitoring the efficacy of an anti-platelet therapy, a method of predicting and/or diagnosing a platelet-related disorder and a method of determining the progression of a platelet-related disorder.

Platelets are one of the key elements of human blood. They play an important role in thrombogenesis, atherogenesis and the progression of atherosclerotic lesions (41-42).

Platelets are not only an important contributor to blood-related diseases, such as those characterised by prolonged bleeding, abnormal platelet activity has also been associated with the pathogenesis of a number of other diseases. For instance, the interaction of platelet with the vessel wall and its subsequent contribution to atheroma formation and thrombosis is of pivotal importance in the aetiology and pathogenesis of peripheral, coronary, cerebrovascular and other vascular diseases (43-44).

Accumulating evidence also suggests a role for platelet activation in cancer progression and an increase in platelet activity has been seen in patients with metastatic cancer (45-47).

Whilst there is increasing realization that inappropriate platelet activation plays a prime role in these diseases, there is still no generally accepted ideal measure of platelet activation that would indicate a state of 'high risk'.

MicroRNAs (miRNAs) are a class of small non-coding RNAs that function as translational repressors. They bind through canonical base pairing to a complementary site in the 3' untranslated region (UTR) of their target mRNAs and can direct the degradation or translational repression of these transcripts. (2, 22) Although the degree of target downregulation by miRNAs tends to be small, miRNAs exert a potent effect on cellular processes due to their ability to control multiple genes that function at different steps in the same biological pathways.(4, 12, 18-19) MiRNAs have been shown to play important roles in development, stress responses, angiogenesis and oncogenesis. (33-34)

Recently, Mitchell *et al.* highlighted the presence of miRNAs in plasma. (20) These plasma miRNAs are not cell-associated, but packaged in microvesicles that protect them from endogenous RNase activity. Interestingly, plasma miRNAs can display unique expression profiles: specific tumour miRNAs were identified in cancer patients (38), while tissue-derived miRNAs constitute a marker for injury (30, 39). Altered levels of plasma miRNAs have been reported in patients with heart failure, coronary artery disease and diabetes (1, 5-6, 29-30, 32).

According to a first aspect of the present invention, there is provided a method of determining platelet activity in an individual comprising determining in a plasma or serum sample obtained from an individual the level of at least one microRNA selected from the group consisting of miR-126, miR-197, miR-223, miR-24 and miR-21.

It has been found that miR-126, miR-197, miR-223, miR-24 and miR-21 are highly expressed in platelets and platelet microparticles. Accordingly, an increased level of miR-126, miR-197, miR-223, miR-24 and/or miR-21 is indicative of increased platelet miRNA content and, therefore, increased platelet activity. A reduced level of miR-126, miR-197, miR-223, miR-24 and/or miR-21 is indicative of decreased platelet miRNA content and, therefore, decreased platelet activity.

Since levels of circulating microRNAs represent the net effect of shedding (microparticles) and clearance (D-dimer, which is a marker for thrombus resolution), an increased level of miR-126, miR-197, miR-223, miR-24 and/or miR-21 is also indicative of an increased level of platelet miRNA shedding into the circulation, a decreased platelet microparticle clearance and/or enhanced thrombus resolution.

In response to acute I/R (ischemia/reperfusion) injury in which the compensation of platelet miroRNA shedding and platelet microparticle clearance may not occur, the levels of all these microRNAs increase and correlate to measurements of platelet microparticles, which are markers of platelet activation.

In diseases, such as diabetes and cardiovascular disease, where a compensatory increase in clearance may actually occur, the levels of the microRNAs may decrease. It has also been found that the baseline levels of these microRNAs (i.e. without I/R injury) correlate to D-dimer, which is a marker of thrombus resolution.

The determination of a high or low level of microRNA is based on a control level, which is typically determined from a relevant population of individuals having normal platelet activities. The relevant population can be defined based on, for example, diet, lifestyle, age, ethnic background or any other characteristic that can affect the normal levels of the markers. Once the control levels are known, the measured levels can be compared and the significance of the difference determined using standard statistical methods. If there is a substantial difference between the measured level and the control level (i.e. a statistically significant difference), then the individual from whom the levels have been measured may be considered to have abnormal platelet activity. By determining the level of platelet activity using the method of the present invention, it is possible to predict whether an individual is at risk of developing a disease which can be characterised by an abnormal level of platelet activity (i.e., a platelet-related disorder). Abnormal platelet activity refers to a level of platelet activity that is either higher or lower than the level in a healthy individual without the disease. In addition, determining the level of platelet activity allows the assessment of the effectiveness of a therapy for such a disease.

According to a second aspect of the present invention, there is provided a method of monitoring the efficacy of an anti-platelet therapy, the method comprising the steps of determining the level of at least one microRNA selected from the group consisting of miR-126, miR-197, miR-223, miR-24 and miR-21 in a plasma or serum sample obtained from an individual before the therapy, determining the level of at least one microRNA selected from the group consisting of miR-126, miR-197, miR-223, miR-24 and miR-21 in a plasma or serum sample obtained from an individual during or after the therapy, and comparing the determined levels in the individual before and during or after the therapy.

Since it was found that miR-126, miR-197, miR-223, miR-24 and miR-21 are highly expressed in platelets and that their levels can be used as an indication of the level of platelet activity in an individual, making the determination set out above allows the efficacy of an anti-platelet therapy to be determined.

As mentioned in the first aspect of the present invention, an increased level of miR-126, miR-197, miR-223, miR-24 and/or miR-21 during or after the therapy is indicative of an increased platelet miRNA content and, therefore, increased platelet activity.

If the anti-platelet therapy reduces platelet microRNA shedding, then a decreased level of miR-126, miR-197, miR-223, miR-24 and/or miR-21 during or after the therapy is a positive indication of the efficacy of the therapy, i.e., a reduction in platelet activity.

On the other hand, if the anti-platelet therapy reduces both platelet microRNA shedding and platelet microparticle clearance, then the change in the levels of the microRNAs depends on the extent to which the shedding and clearance are reduced.

An increased or decreased level of a microRNA after or during the therapy means that the level of the microRNA obtained after or during the therapy is higher or lower than that obtained before the therapy, respectively.

According to a third aspect of the present invention, there is provided a method of predicting and/or diagnosing a platelet-related disorder comprising determining in a plasma or serum sample obtained from an individual the level of at least one microRNA selected from the group consisting of miR-126, miR-197, miR-223, miR-24 and miR-21, and comparing the determined level in the individual with a control level.

Since it was found that miR-126, miR-197, miR-223, miR-24 and miR-21 are highly expressed in platelets and that their levels vary according to the activation state of the platelets, the level of these mircroRNAs can be used as an indication or biomarker of the level of platelet activity of an individual, thereby predicting or diagnosing a platelet-related disorder.

The term "a platelet-related disorder" refers to a disease or condition involving abnormal platelet activity, which may be higher or lower than the level of platelet activity in a healthy individual without the disorder. Platelet-related disorders include, but are not limited to, von Willebrand disease, Bernard-Soulier syndrome, Glanzmann thrombasthenia, thrombocytopenia, Henoch-Schönlein Purpura, thrombotic thrombocytopenic purpura, hemolytic uremic syndrome, acquired aplastic anemia, Wiskott-Aldrich syndrome, grey platelet syndrome, cancer and leukemia. Preferably, a platelet-related disorder does not include diabetes, vascular diseases or cardiovascular diseases, although abnormal platelet function is known to occur.

By making the determination set out above, it is possible to determine with high specificity and sensitivity whether an individual has or is likely to develop a platelet-related disorder. Specificity is defined as the proportion of true negatives (individuals that do not have or do not develop a platelet-related disorder) identified as such in the method. Sensitivity is defined as the proportion of true positives (individuals that have or are likely to develop a platelet disorder) identified as such in the method. The method provides a highly accurate test that can be performed relatively easily using any of the biomarkers.

Based on the nature of the platelet activity (mentioned above) in a particular platelet-related disorder, the levels of the microRNAs in an individual having, or likely to develop, the platelet-related disorder may be higher or lower than a control level.

For a platelet-related disorder characterised by an abnormally high platelet miRNA content, increased platelet miRNA shedding into the circulation, decreased platelet microparticle clearance and/or enhanced thrombus resolution (i.e., increased platelet activity), an increased level of miR-126, miR-197, miR-223 and/or a miR-24 is a positive indication of the disorder or the likelihood of developing such a disorder.

For a platelet-related disorder characterised by an abnormally low platelet miRNA content, decreased platelet miRNA shedding into the circulation, increased platelet microparticle clearance and/or impaired thrombus resolution (i.e., decreased platelet activity), a decreased level of miR-126, miR-197, miR-223 and/or miR-24 is a positive indication of the disorder or the likelihood of developing such a disorder.

The method according to the third aspect of the present invention allows the identification of individuals with a platelet-related disorder. The method also allows the identification of individuals that are likely to develop a platelet-related disorder. The method therefore enables preventative action to be taken, such as changes to the diet and lifestyle of the individual, as well as medical intervention.

According to a fourth aspect of the present invention, there is provided a method of monitoring the progression of a platelet-related disorder comprising the steps of determining the level of at least one microRNA selected from the group consisting of miR-126, miR-197, miR-223, miR-24 and miR-21 in a plasma or serum sample obtained from an individual at a first time point, determining the level of at least one microRNA selected from the group consisting of miR-126, miR-197, miR-223, miR-24 and miR-21 in a plasma or serum sample obtained from an individual at a second time point, and comparing the determined levels in the individual at the first and second time points.

The second time point is after the first time point and the two time points are sufficiently spaced to allow the status of the disorder to change in such a manner so as to allow progression of the disorder to be monitored.

Since it was found that the levels of miR-126, miR-197, miR-223, miR-24 and miR-21 can be used as an indication of the level of platelet activity in an individual, making the determination set out above allows the progression of a platelet-related disorder to be monitored.

Based on the nature of the platelet activity (mentioned above) in a particular platelet-related disorder, progression of the platelet-related disorder may be indicated by higher or lower levels of the microRNAs.

For a platelet-related disorder characterised by an abnormally high platelet miRNA content, increased shedding of platelet miRNAs into the circulation, decreased platelet microparticle clearance and/or enhanced thrombus resolution (i.e., increased platelet activity), an increased level of miR-126, miR-197, miR-223, miR-24 and/or miR-21 is a positive indication of the progression of the platelet-related disorder.

For a platelet-related disorder characterised by an abnormally low platelet miRNA content, decreased shedding of platelet miRNAs into the circulation, increased platelet microparticle clearance and/or impaired thrombus resolution (i.e., decreased platelet activity), a decreased level of miR-126, miR-197, miR-223, miR-24 and/or miR-21 is a positive indication of the progression of the platelet-related disorder.

An increased or decreased level of a microRNA means that the level of the microRNA obtained at the second time point is higher or lower than that obtained at the first time point, respectively.

The methods according to all aspects of the present invention are performed on a plasma or serum sample obtained from an individual. The sample may be any suitable sample from which it is possible to measure the microRNAs mentioned above.

miR-21 is a standard term well known to those skilled in the art. In particular, the sequence of the human form of miR-21 is given in the NCBI protein database under accession number NR_029493.1, version GI: 262205659.

miR-24 is a standard term well known to those skilled in the art. In particular, the sequence of the human form of miR-24 is given in the NCBI protein database under accession number, NR_029496.1, version GI:262205676.

miR-126 is a standard term well known to those skilled in the art. In particular, the sequence of the human form of miR-126 is given in the NCBI protein database under accession number NC_029695.1, version GI: 262205369.

miR-197 is a standard term well known to those skilled in the art. In particular, the sequence of the human form of miR-197 is given in the NCBI protein database under accession number NC 029583.1, version GI: 262206094.

miR-223 is a standard term well known to those skilled in the art. In particular, the sequence of the human form of miR-223 is given in the NCBI protein database under accession number NC_029637.1, version GI: 262206350.

miR-150 is a standard term well known to those skilled in the art. In particular, the sequence of the human form of miR-150 is given in the NCBI protein database under accession number NR_029703.1, version GI: 262205410.

miR-93 is a standard term well known to those skilled in the art. In particular, the sequence of the human form of miR-93 is given in the NCBI protein database under accession number NR_029510.1, version GI: 262205737.

For the avoidance of doubt the specific sequences of the markers mentioned above are defined with respect to the version present in the database at the priority date of the present application. The specific sequences of the markers are exemplary. Those skilled in the art will appreciate that polymorphic variants exist in the human population and that the identification of such polymorphic variants is standard practice to those skilled in the art.

There are numerous ways of determining the level of the microRNAs, including Northern blotting, microRNA arrays, real-time RT-PCR methods, next generation sequencing, differential display, RNA interference, RNase protection methods, etc. Such methods are well known to those skilled in the art (see for example Ach et al., BMC Biotechnology, 8, 69, 2008). Preferably the levels of the microRNAs are measured using real-time RT-PCR methods.

In some embodiments, the levels of a plurality of microRNAs selected from the group consisting of miR-126, miR-197, miR-223, miR-24 and miR-21 are determined. For example, the levels of two, three, four or all five of the microRNAs are determined.

Since these microRNAs are expressed in different cell types (i.e. not exclusively in platelets), by determining the levels of more than one microRNA and their relationship to each other, one can get more reliable information on platelet activity.

In some embodiments, the levels of at least two microRNAs selected from the group comprising miR-126, miR-197 and miR-223 or miR-24 are determined. Preferred combinations of three microRNAs are shown in Table 5 below.

In particular embodiments, the levels of i) miR-126, miR-197 and miR-223; ii) miR-126, miR-197 and miR-24; or iii) miR-126, miR-24 and miR-150; iv) miR-126, miR-miR-223 and miR-93; or v) miR-126, miR-197, miR-223 and miR-24 are determined. Preferably, the levels of i) miR-126, miR-197 and miR-223; or ii) miR-126, miR-197 and miR-24, are determined.

The present invention will now be described in detail by way of example only with reference to the following figures.
Figure 1A shows the pairwise correlations between circulating miRNAs
Figure 1B shows the circulating miRNA signature for incident MI. L₁-penalized Cox regression analysis (least absolute shrinkage and selection operator method). The graph shows Cox regression coefficients of miRNAs (y-axis) for different levels of penalization (the tuning parameter λ₁ escalates beyond 20). Variables withstanding shrinkage up to high λ₁ values are those most relevant for disease prediction.
Figure 2 shows risk estimates for the three miRNAs most consistently associated with MI. The three miRNAs were identified by AIC-based models and the technique of least absolute shrinkage and selection operator. Hazard ratios (95% CI) were derived from standard Cox regression models with progressive adjustment. Expression levels were normalised to U6 expression.
Figure 3 shows the interventional study to identify miRNA clusters. A. Model of limb I/R injury in healthy volunteers. Arrows indicate timepoints of sample collection. B. MiRNA clusters. Temporal clustering by affinity propagation grouped the 30 miRNAs into 6 distinct clusters.
Figure 4 shows the miRNA cluster associated with MI. Cluster 2 contained all miRNAs associated with MI. Temporal changes were assessed by QPCR. Exogenous spike-in cel-miR-39 was used as a normalization control. Red bars indicate statistically significant differences (p<0.05) compared to baseline.
Figure 5 shows miRNA expression in the IR cohort at baseline and at different time points, as assessed by QPCR. Exogenous spike-in cel-miR-39 was used as a normalization control. Red bars indicate statistically significant differences (p<0.05) compared to baseline.

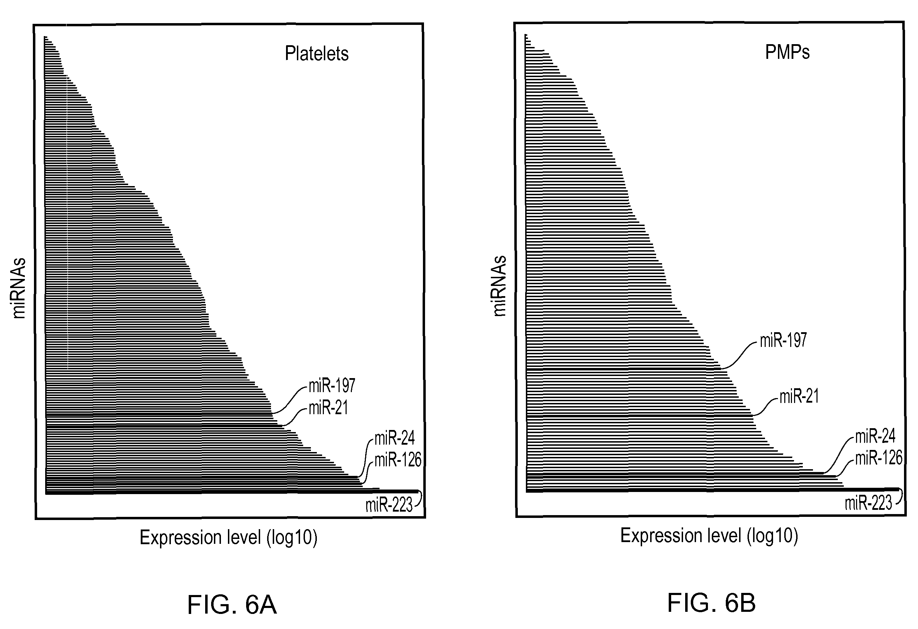

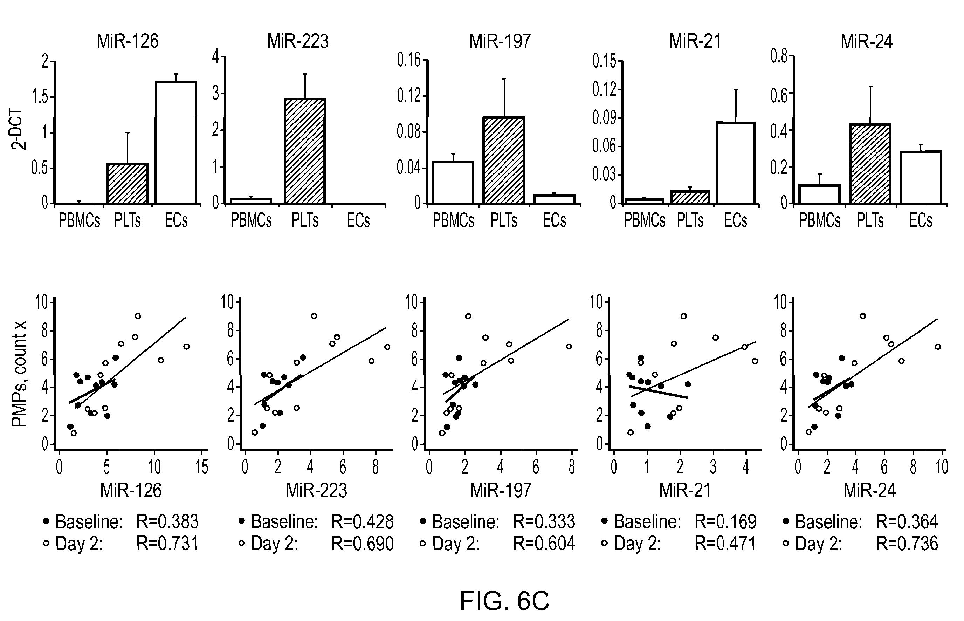

Figure 6 shows the platelet contribution to miRNA signature for MI. (A) MiRNA profile of platelets, as determined by microarray screening. The average Ct value was used as a normalization control. Expression levels (2^{-DCt}) were log transformed. (B) Corresponding miRNA profile of platelet microparticles (PMPs). miRNAs associated with MI are abundant in platelets and platelet microparticles. (C) Cell type-specific miRNA expression. Comparison of miR-126, miR-223, miR-197, miR-21 and miR-24 expression in peripheral blood mononuclear cells (PBMCs), platelets (PLTs) and endothelial cells (ECs) using QPCR (upper panel). Raw Ct values were normalized to U6 expression (means ± SD, n=3). Correlation of miRNA levels with PMP counts (lower panel) at baseline (black dots) and 2 days post I/R injury (grey dots). R-values are Pearson correlation coefficients (N=11, P<0.05 for R>0.602).
Figure 7 shows the top 20 three miRNA combinations plotted based on the derived AIC and IDI values shown in Table 5 below.
Figure 8 shows the level of miRNAs in plasma samples obtained from healthy patients and patients with symptomatic carotid stenosis receiving anti-platelet therapy.

### EXAMPLE

### Material and Methods

**Study subjects**. The Bruneck Study is a prospective population-based survey initiated in 1990 as a sex- and age-stratified random sample of all inhabitants of Bruneck 40 to 79 years old (125 women and 125 men in the fifth to eighth decades each)(15). The current analysis focuses on 820 participants of the 1995 evaluation for whom RNA extracted from blood specimen was available. Follow-up in 2000 and 2005 was 100% complete for clinical endpoints and > 90% complete for repeated laboratory examinations. Fatal and non-fatal MI was ascertained following the WHO criteria for definite disease status (11). Diabetes was coded present if the individual had a fasting glucose ≥ 140 mg/dL, a two-hour glucose level ≥ 200 mg/dL after 75g glucose load or a pre-established record-confirmed diagnosis of diabetes. Systolic and diastolic blood pressure was taken with a standard mercury sphygmomanometer after at least ten minutes of rest (mean of three independent measurements). LDL cholesterol was assessed using the Friedewald equation (9). To explore the origin of circulating miRNAs, healthy volunteers (n=11, 19-51 yr) underwent limb I/R generated by thigh cuff inflation. Plasma samples were taken at baseline, at 10 min, 1h, 5h, 2 days and 7 days following injury. The protocols of both studies were approved by the appropriate ethics committees and all study subjects gave their written informed consent before entering the study.

**RNA isolation, reverse transcription and pre-amplification.** Circulating miRNAs were extracted using the miRNeasy kit (Qiagen) as described previously (32). A fixed volume of 3 µl of the 25 µl RNA eluate was used as input in each reverse transcription (RT) reaction. An RT reaction and pre-amplification step were performed as described previously (32). In brief, miRNAs were reversely transcribed using Megaplex Primer Pools (Human Pools A v2.1, Applied Biosystems). RT reaction products were further amplified using the Megaplex PreAmp Primers (Primers A v2.1) as recommended by the manufacturer. Both RT and PreAmp products were stored at -20°C.

**Taqman qPCR assay.** Taqman miRNA assays were used to assess the expression of individual miRNAs. 0.5 µl diluted pre-amplification product were combined with 0.25 µl Taqman miRNA Assay (20x) (Applied Biosystems) and 2.5 µl Taqman Universal PCR Master Mix No AmpErase UNG (2x) to a final volume of 5 µl. QPCR was performed on an Applied Biosystems 7900HT thermocycler at 95°C for 10 min, followed by 40 cycles of 95°C for 15 sec and 60°C for 1 min. All samples were run in duplicates. Relative quantification was performed using the software SDS2.2 (Applied Biosystems). U6 expression was used for normalization purposes. In a second round, the average Ct value of all miRNAs was applied as an additional normalization control. This commonly used approach is expected to results in weaker associations because levels of individual miRNAs and their clusters contribute to the Ct average.

**MiRNA screening in platelets and platelet microparticles.** Platelets were isolated from healthy subjects (23). In brief, blood was drawn using acid citrate dextrose as anticoagulant (ACD: 120 mmole/L sodium citrate, 110 mmole/L glucose, 80 mmole/L citric acid, 1:7 vol/vol) and centrifuged for 17 minutes at 200g and 30°C in the presence of indomethacin (10 µmole/L; Sigma-Aldrich). The platelet-rich plasma was then centrifuged for another 10 minutes at 1000*g* in the presence of prostacyclin (0.1 µg/mL; Sigma-Aldrich). The resulting platelets were resuspended in modified Tyrode-HEPES buffer (145 mmole/L NaCl, 2.9 mmole/L KCl, 10 mmole/L HEPES, 1 mmole/L MgCl₂, 5 mmole/L glucose, pH 7.3) at a concentration of 4 x 10⁸/mL. Platelet microparticles were isolated following platelet activation with thrombin (0.1 U/mL; Sigma-Aldrich). Platelet aggregation was monitored with a turbidometric method (Chronolog 490; Chronolog). Platelet microparticles were harvested by ultracentrifugation at 100000g for 90 minutes at 4°C. The pellet was lysed in Qiazol reagent and RNA was extracted as described above. Total RNA was eluted in 25 µl of nuclease free H₂O. RNA was quantified using the NanoDrop spectrophotometer and 20 ng of total RNA were used for reverse transcription. The expression profile of platelets and platelet microparticles was assessed using the Human Taqman miRNA Array Card A (Applied Biosystems) as previously described (32).

**Other cell types.** Peripheral blood mononuclear cells (PBMCs) were isolated according to standard protocol. Heparinized whole blood (5 to 8 mL) was diluted to 10 mL with phosphate-buffered saline (PBS) (pH 7.4), layered on top of 5 mL Histopaque 1083, and centrifuged for 30 minutes at 400g. PBMCs were washed twice, resuspended in PBS, and counted with a hemocytometer. Human umbilical vein endothelial cells (ECs) were purchased from Cambrex and cultured on gelatin-coated flasks in M199 medium supplemented with 1 ng/mL endothelial cell growth factor (Sigma), 3 µg/mL endothelial growth supplement from bovine neural tissue (Sigma), 10 U/mL heparin, 1.25 µg/mL thymidine, 10% foetal bovine serum, 100 µg/mL penicillin and streptomycin. The cells were subcultured every 3 days to a ratio 1:4.

**Statistical analysis.** Data were analyzed using the statistical packages SPSS version 15.0 and STATA version 10.1. Normally distributed continuous variables were presented as means ± standard deviation (SD), variables with a skewed distribution as geometric means ± geometric SD, and dichotomous variables as numbers and percentages. The T-test and Fisher's exact test were used to analyze differences in participant characteristics between those who developed MI during follow-up and those who did not. Logₑ-transformed miRNAs were used for all computations to approximate a Gaussian distribution. Correlations between miRNAs were assessed using Pearson correlation coefficients with Bonferroni-adjusted P-values. Cox proportional hazard regression models were fitted to assess the association between loge-transformed miRNA levels and incident MI. To identify the subset or pattern of miRNA with the highest prognostic ability for future MI, two distinct approaches were used: (1) The first one was a two-step procedure. In order to reduce the number of candidate miRNAs and subsequent computational requirements forward and backward stepwise Cox regression analyses with relaxed in- and exclusion criteria (P_{entry}=0.15 and Pᵣₑₘₒᵥₐₗ=0.20; adjusted for age, sex and previous cardiovascular disease) were fitted. Seven miRNAs consistently detectable in the circulation were selected in either or both of the analyses (miR-24, miR-126, miR-140, miR-150, miR-197, miR-223, and miR-486) and considered eligible for the second 'best subset' step. Cox regression models of all combinations of eligible miRNAs were computed and compared according to the models' Akaike information criterion (AIC) that is based on the maximized log-likelihood and imposes a penalty for increasing the number of parameters in the model. Lower values of AIC indicate the preferred model which is the one with the fewest parameters still providing adequate fit (tradeoff between accuracy and complexity). (2) The second approach utilized the technique called L₁-penalization implementing the '**l**east **a**bsolute **s**hrinkage and **s**election **o**perator [lasso] algorithm' to all 19 miRNAs. L₁-penalized methods shrink the estimates of the regression coefficients towards zero relative to the maximum likelihood estimates. The technique has been employed to generate gene signatures from microarray data and prevents overfit arising from both co-linearity and high-dimensionality. The amount of shrinkage is determined by the tuning parameter λ₁, which is progressively increased up to the value that shrinks all regression coefficients to zero. Plots of fitted regression coefficients (y-axis) versus λ₁ (x-axis) were generated using the 'penalized' package of R statistical software (10). The lasso method allows assessing the relevance and robustness of individual explanatory variables but produces biased estimated for the regression coefficients. Accordingly, risk estimates for the three miRNAs finally selected (both approaches identified the same miRNA signature) were computed by standard Cox regression analysis and adjusted for age and sex, smoking status (ever vs. never smokers), systolic blood pressure, LDL cholesterol, diabetes and history of cardiovascular disease (multivariable model 1), plus other miRNAs (multivariable model 2), plus body mass index, waist-hip ratio, HDL cholesterol, logₑ C-reactive protein and fibrinogen (multivariable model 3). In the interventional study, miRNA expression profiles were clustered using the Temporal Clustering by Affinity Propagation algorithm (14). This is an efficient and parameter-free approach for identification of temporal features in expression datasets, and has previously been shown to produce biologically meaningful groupings.

Two-sided P values below 0.05 were considered significant.

### Statistics for the top 20 miRNA combinations tested.

All combinations of eligible miRNAs were computed and compared according to the models' *Akaike information criterion (AIC)* that is based on the maximized log-likelihood and imposes a penalty for increasing the number of parameters in the model. See Table 5. Lower values of AIC indicate the preferred model which is the one with the fewest parameters still providing adequate fit (tradeoff between accuracy and complexity). Moreover, the inventor examined the discrimination of the models with and without miRNA by using the *Harrell c-index* (Harrell et al., Evaluating the yield of medical tests. JAMA. 1982, 247:2543-6; Pencina MJ et al., Stat Med. 2004, 23:2109-23; Liu L et al., Fitting Cox model using PROC PHREG and beyond in SAS. Presented at SAS Global Forum 2009, Washington, DC, 22-25 March 2009. Paper no. 236-2009). The *net reclassification improvement (NRI)* formula (Pencina MJ et al., Stat Med. 2008, 27:157-72; Steyerberg EW et al., Ann Intern Med. 2010, 152:195-6.5, 6) was used to assess the extent to which adding information on miRNAs reassigns participants to risk categories that better reflect their cardiovascular disease outcome (10-year risk categories < 10%, 10% to 20%, and >20%). Approximate 95% CIs for the NRI were calculated by using the same variance terms as in the test of significance (Armitage P. Statistical Methods in Medical Research. Oxford: Blackwell Scientific; 1977). Unlike the NRI, the calculation of the *integrated discrimination improvement (IDI)* does not rely on arbitrarily chosen cut-offs for 10-year risk categories.

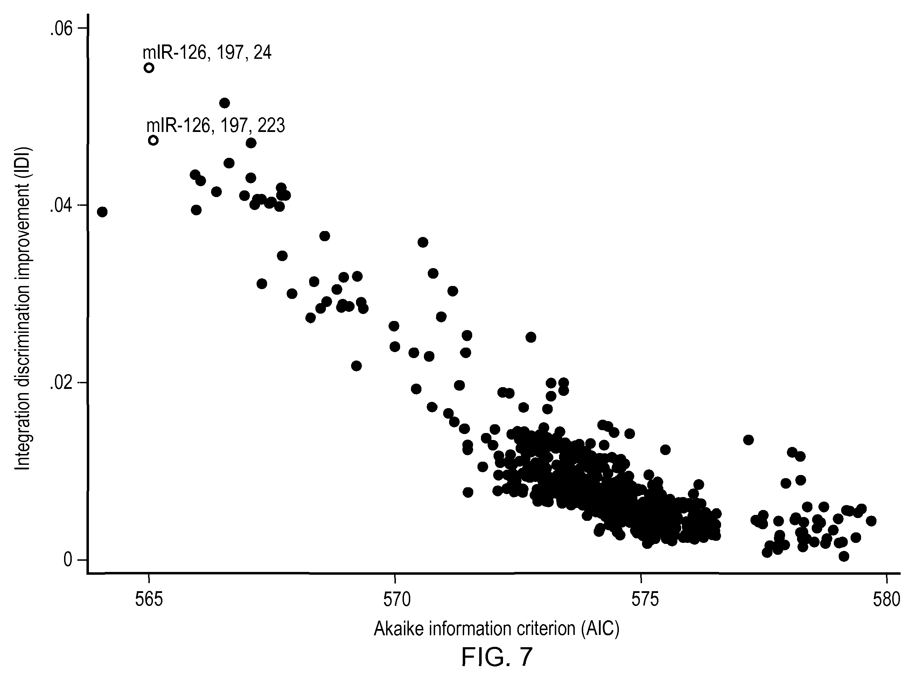

The combinations of all miRNAs were plotted based on their improvement of the AIC and the IDI to demonstrate that the combinations of miR-126, miR-197 and miR-223 or miR-24, are the best among all miRNA combinations tested. Note that miR-223 and miR-24 are highly correlated and thus generally interchangeable. See Figure 7.

### Pharmacological intervention study in healthy volunteers.

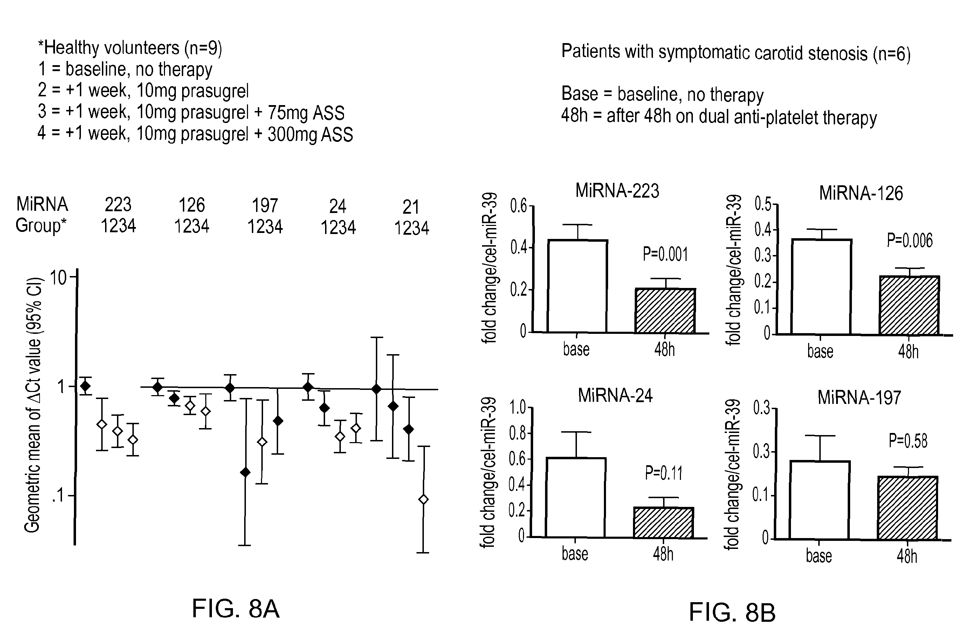

Healthy young males (<30 yrs) were given 10 mg prasugrel (active metabolite of clopidogrel, week 1), followed by a combination therapy with low dose aspirin (75mg, week 2) and high dose aspirin (300 mg, week 3). This dose escalation of aspirin in combination with prasugrel (dual anti-platelet therapy) results in increasing platelet inhibition. Plasma samples were obtained at each study visit. MiR-223, miR-197, miR-126, miR-24 and miR-21 were measured by qPCR. A reduction in these platelet miRNAs was observed with increasing platelet inhibition. See Figure 8.

These findings in healthy volunteers were corroborated by miRNA measurements in patients with symptomatic carotid stenosis participating in a randomized trial to determine whether treatment with dipyridamole or clopidogrel, in addition to aspirin, was more effective at reducing embolization. Treatment efficiency was evaluated using transcranial Doppler detection of embolic signals and platelet aggregometry at baseline and 48 hours. Both treatment regimens had similar efficacy in reducing embolization. Levels of platelet miRNAs were measured before and after anti-platelet therapy. See Figure 8.

### RESULTS

**Circulating miRNAs in the Bruneck Study.** Baseline demographic, clinical and laboratory characteristics of the 820 participants in the 1995 evaluation are shown in Table 1. All subjects were of Caucasian origin. A total of 47 participants experienced myocardial infarction over the 10-year follow-up period, corresponding to an incidence rate of 6.5 [95% CI 4.9-8.6] per 1000 person-years. For the initial screening, Human Taqman miRNA arrays (CardA v2.1 and CardB v2.0, Applied Biosystems) covering 754 small non-coding RNAs were applied to 8 pooled samples, consisting of subjects with and without atherosclerotic vascular disease matched for different cardiovascular risk factors (hyper-cholesterolemia, smoking, hypertension, diabetes) as described previously (32). Of the 148 miRNAs with Ct-values <36, 130 miRNAs were detected using fluidic CardA and therefore all further analysis focused on this dataset. The network analysis revealed seven miRNAs as promising targets that were reliably detectable in the circulation (32). These were selected and measured in the entire Bruneck cohort (n=820). An additional twelve miRNAs were quantified as part of an ongoing project on osteoarthritis (n=820). MiRNA levels were strongly correlated with each other, with some reaching almost perfect correlation e.g. miR-24 and miR-223, r=0.939. The complex dependency of miRNAs in participants who did and did not suffer MI was further scrutinized as miRNA-miRNA correlation profiles.

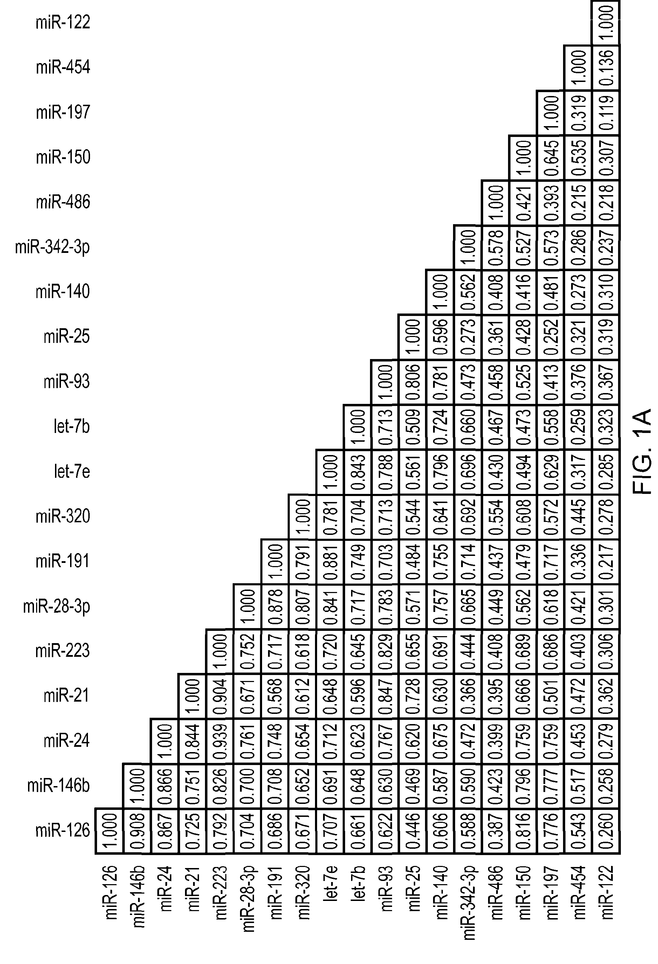

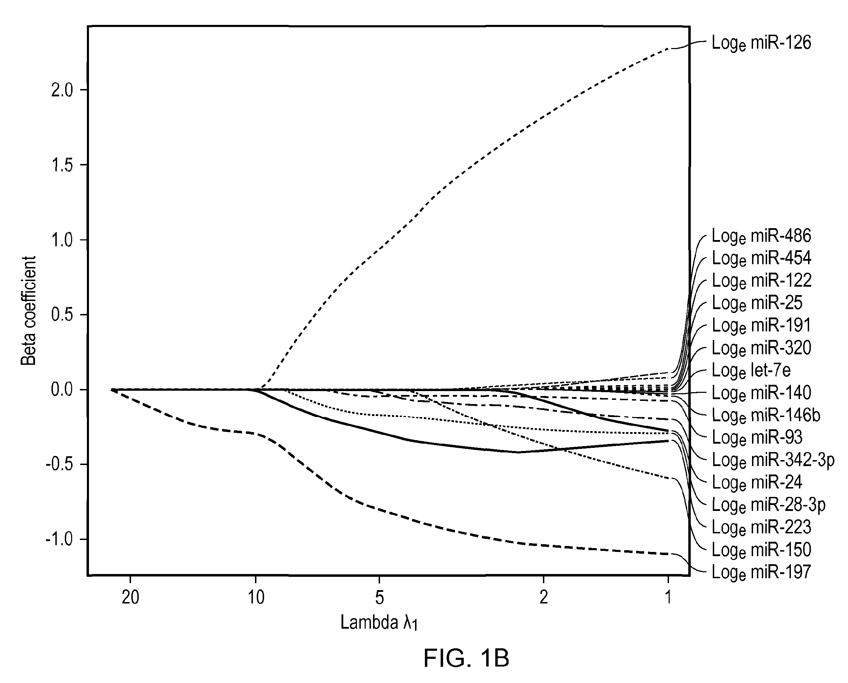

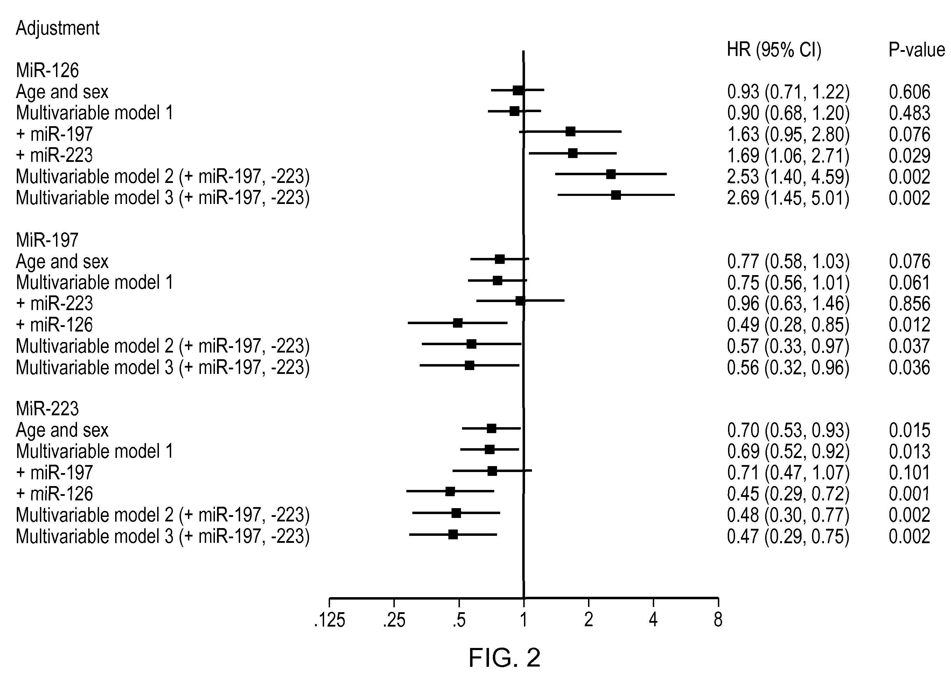

**Association with incident MI.** MiRNAs associated with incident MI were selected using two different approaches: (1) Stepwise Cox regression with comparison of AIC, a criterion considering both goodness of fit and the number of parameters in the model, identified two preferred combinations of miRNAs: miR-126/-197/-223 and miR-126/-197/-24 (AIC ~ 563 each with 6 degrees of freedom). (2) In L₁-penalized Cox regression analysis, miR-126, miR-197 and miR-223 showed the strongest association with incident MI at any level of penalization (λ₁) and emerged as the miRNAs requiring the highest λ₁ for their regression coefficients to be shrunk to zero (Figure 1B) whereas miR-24 performed worse. Accordingly, the inventors gave preference to the miR-126/-197/-223 combination excluding miR-24. MiR-126 showed positive association with MI, while miR-197 and miR-223 were inversely related to disease risk (Figure 2). Similar results were also obtained following normalization to the average Ct value of all assessed miRNAs. There was no effect modification by sex, diabetes or pre-existing cardiovascular disease (Table 2A and 2B (Table 2B is an enhanced version of Table 2A wherein the normalisation of the data has been corrected). When the other miRNAs were individually added to the multivariable model already including miR-126/-197/-223, none achieved statistical significance. In subanalysis, miR-223 showed a stronger association with fatal than non-fatal MI but was equally predictive for early (1995-2000) and late events (2000-2005) (data not shown). In contrast, miR-126 and miR-197 were only predictive for early events (data not shown). These findings were similar for normalization to U6 or the average Ct value of all miRNAs.

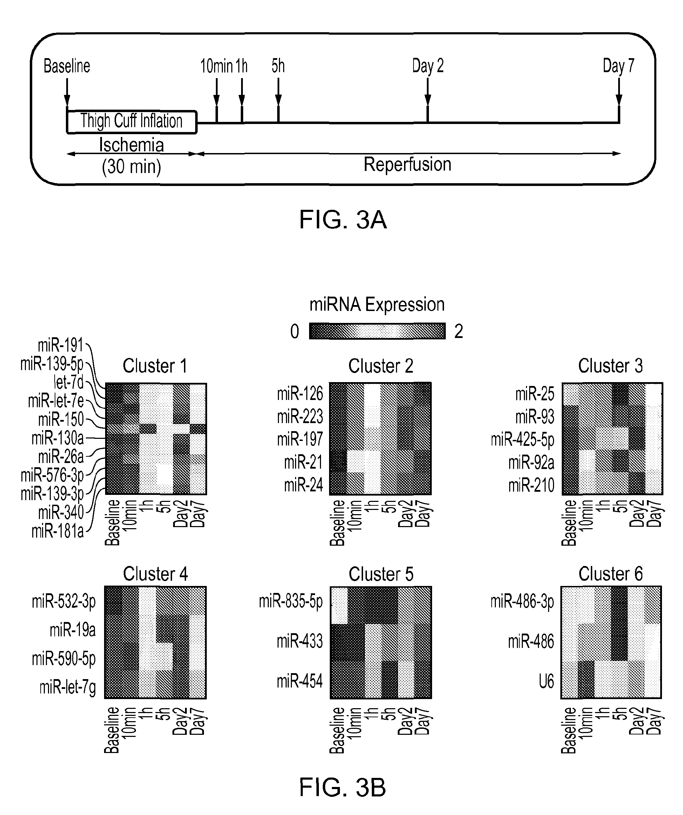

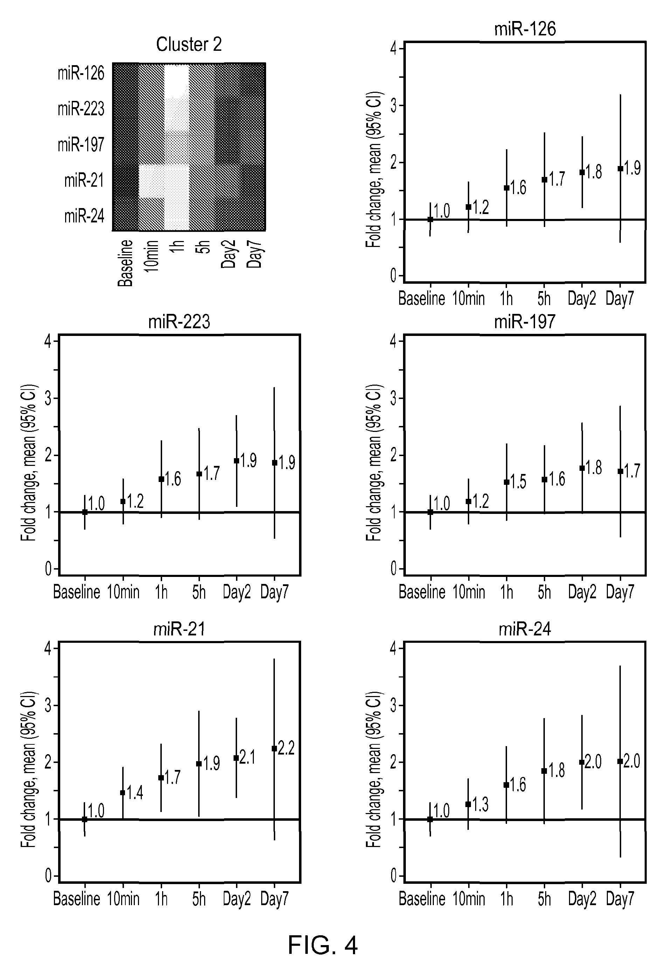

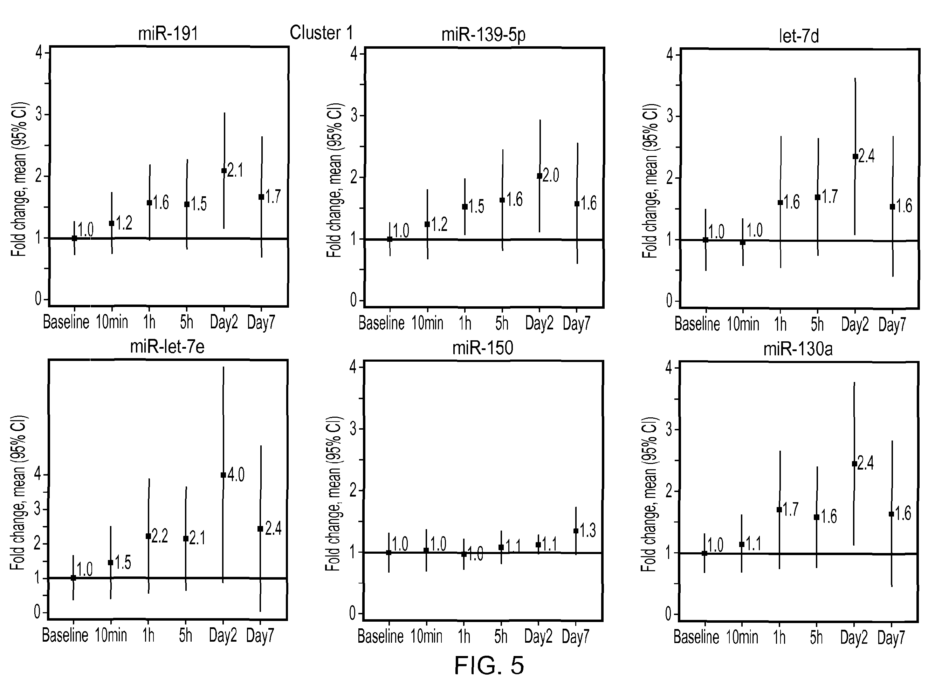

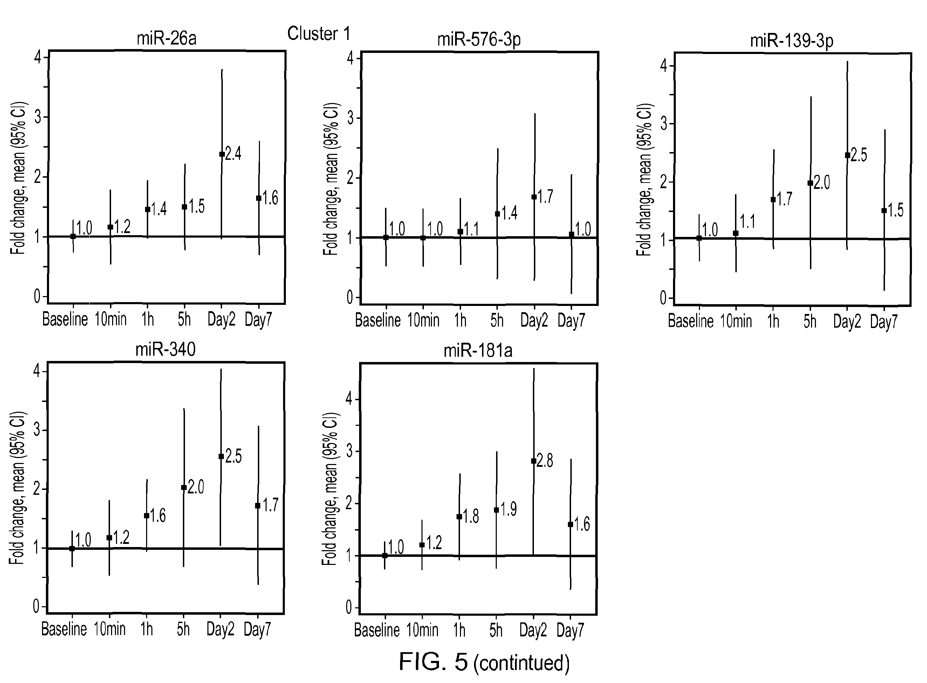

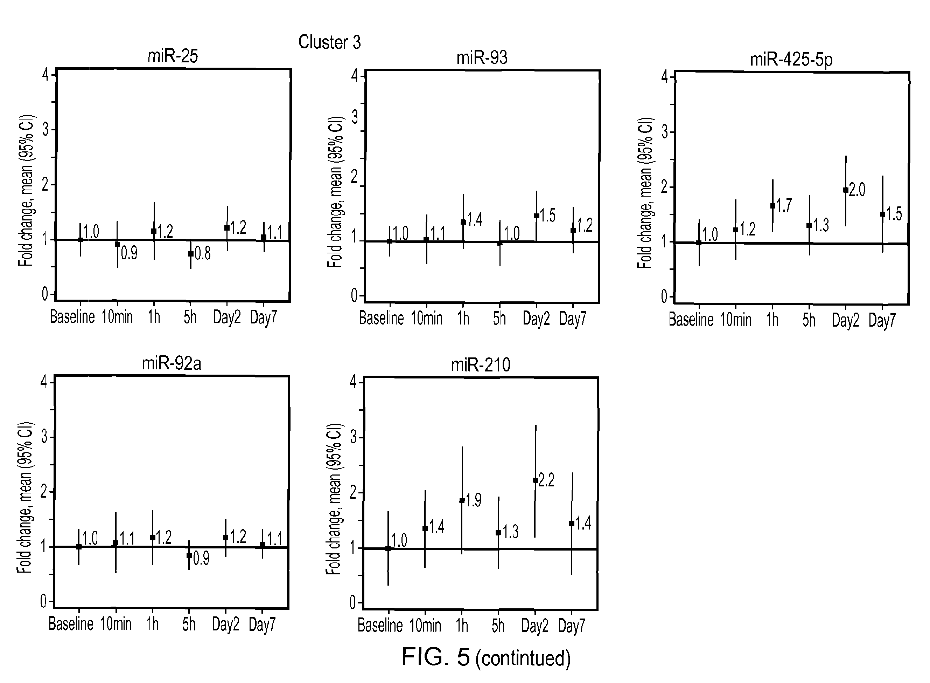

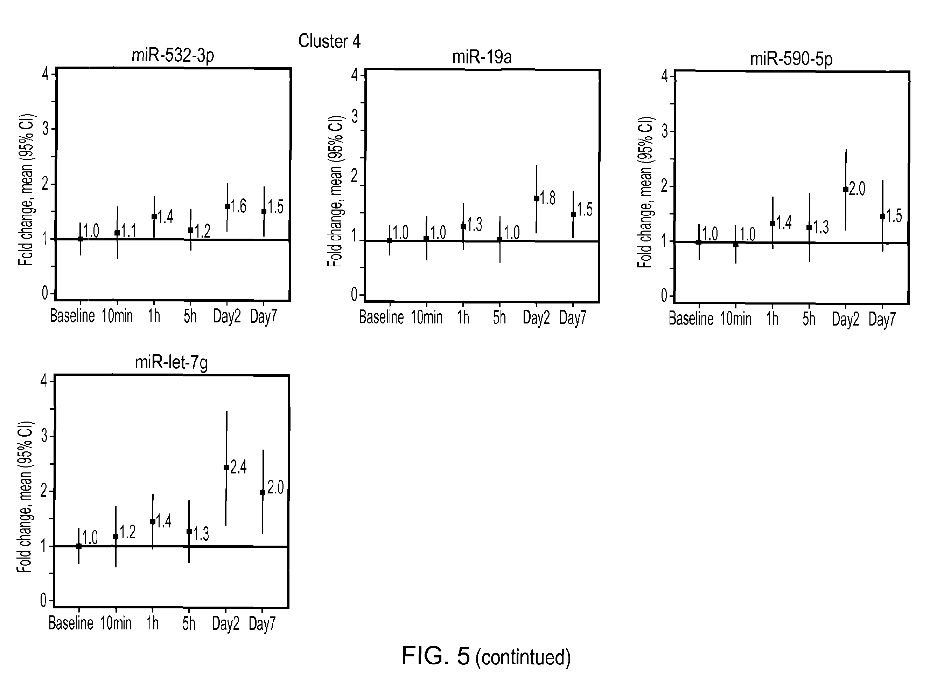

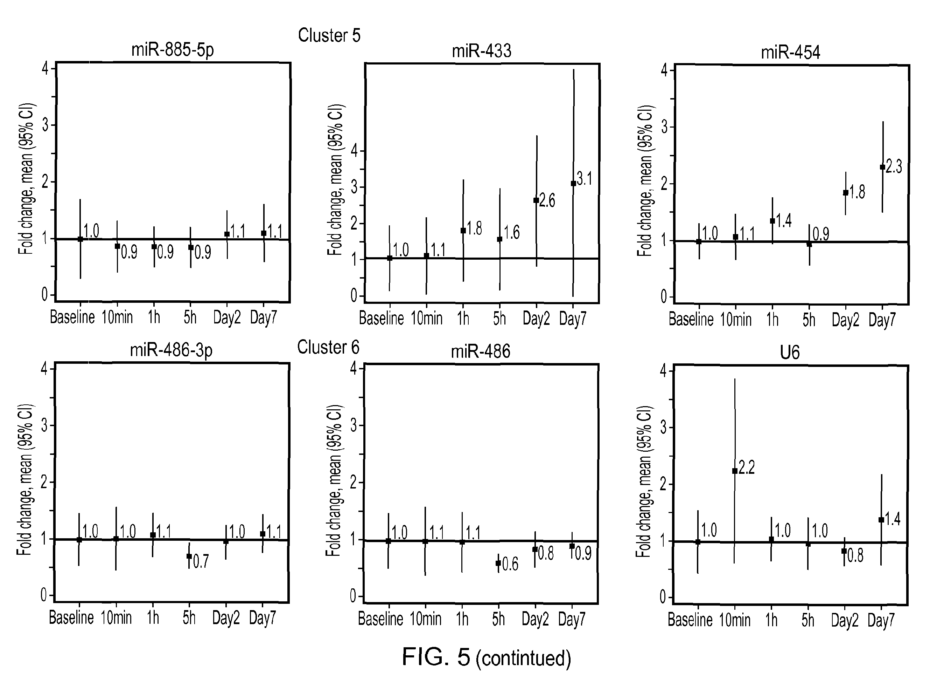

**Interventional study.** To explore the cellular origin of circulating miRNAs, healthy volunteers (n=11) underwent limb I/R generated by cuff inflation. Plasma samples were collected at baseline and 10 min, 1h, 5h, 2 days and 7 days following injury (Figure 3A). The clinical characteristics of the study population are shown in Table 3. Thirty miRNAs, including the miRNAs associated with MI in the Bruneck study, were assessed by QPCR. Computational analysis using the Temporal Clustering by Affinity Propagation algorithm based on average miRNA expression at baseline and over time identified 6 distinct miRNA clusters (Figure 3B). Cluster 2 was of particular interest: all miRNAs associated with future MI (miR-126, miR-223, miR-197, and miR-24) were part of to this cluster characterised by an early increase at 1h and sustained elevation until 7 days post I/R injury (Figure 4). MiR-21 was also included. Results were robust with different methods of normalization (exogenous spike-in cel-miR-39 control and unadjusted Ct values). The response of other miRNAs to I/R injury is shown in Figure 5. Significant correlations of baseline microRNA plasma levels with D-dimer, a fibrin degradation product and marker of endogenous fibrinolysis, are highlighted in Table 4.

**Platelet contribution.** I/R injury induces platelet activation (33, 34) and platelets have recently been shown to contain miRNAs. (2, 22) To test for a potential platelet contribution to the observed miRNA changes after I/R, miRNA screening was performed in preparations of platelets and platelet microparticles derived from thrombin-activated platelets. The miRNAs constituting our cluster of interest (miR-223, -126, -24, -21, and -197) were among the most abundant miRNAs in platelets (Figure 6A) and platelet microparticles (Figure 6B). A comparison with peripheral blood mononuclear cells (PBMCs) and endothelial cells (ECs) confirmed that miR-223 and miR-24 were predominantly found in platelets (PLTs), while miR-197 was also present in PBMCs. In contrast, miR-126 and miR-21 were highly enriched in endothelial cells, but detectable in platelets, albeit at a much lower concentrations (Figure 6C, upper panel). The platelet origin of these circulating miRNAs was further supported by a significant correlation (except miR-21) with PMP counts at 2 days post I/R injury (Figure 6C, lower panel). In comparison, no association was obtained for endothelial and erythrocyte microparticles (data not shown). Hence, adjustment for platelet-derived miRNAs may refine the endothelial contribution to the miR-126 content in the circulation. This is in agreement with our results from the Bruneck study, where adjustment for miR-197 and miR-223 exposed the opposing directionality of changes in miR-126 in participants with subsequent MI (Figure 2).

The data in Table 5 of the top 3 miRNA combinations demonstrates that various combinations of markers are useful in determining the level of platelet activation. In particular, the data in Figure 7 shows that best combinations are: i) miR-126, miR-197 and miR-223; and ii) miR-126, miR-197 and miR-24.

The data provided also demonstrates that the levels of the miRNAs decrease when healthy volunteers or patients receive anti-platelet therapy. Accordingly, the levels of the miRNAs can be used to monitor the effectiveness of anti-platelet therapy.

### DISCUSSION

**MiRNAs as biomarkers for MI.** In the analysis, the inventors considered seven miRNAs that emerged as promising targets for cardiovascular disease in the pre-screening and displayed unique network topology (32). Three of these miRNAs formed part of a signature for MI: miR-126, miR-197 and miR-223. The findings were independent of classic vascular risk factors, stable in subgroups (men and women, diabetics and non-diabetics, participants with and without previous cardiovascular disease) and robust when using distinct statistical approaches. Another twelve miRNAs were not related to atherosclerotic vascular disease in the pre-screening and fell short of significance in the main analysis. MiR-223 is considered to be a myeloid specific miRNA that acts as a fine-tuner of granulocyte production and the inflammatory response (13, 24, 27). MiR-197 has been reported as differentially expressed in tumours (17, 26). In contrast, miR-126 is highly enriched in endothelial cells and facilitates VEGF signalling (8). However, little is known about the cellular origin of these miRNAs in the circulation.

**MiRNA response after IR injury.** Based on expression profiles after IR injury, miR-126, miR-197 and miR-223 were part of one cluster that also included miR-21 and miR-24. All these miRNAs are highly expressed in platelets and platelet microparticles. This is, to our knowledge, the first time that the contribution of a specific cell type to circulating miRNAs has been defined by a controlled intervention. The present findings extend our previous observations in patients with diabetes (32) and raise the possibility that the observed loss of several miRNAs, including miR-126, miR-197, miR-223, miR-24 and miR-21, may reflect abnormal platelet function in diabetic patients. A reduction of platelet miRNAs could reflect a decrease in the miRNA content of platelets, increased clearance of platelet microparticles by inflammatory cells or impaired thrombus resolution. In contrast, changes of miR-126 in participants with future MI did not follow the same directionality as miR-197 and miR-223, arguing against a common platelet origin. Higher levels of circulating miR-126 could be in line with previous reports implicating endothelial microparticles as biomarkers for vascular damage and increased cardiovascular risk (25). Regardless, our findings advice caution in interpreting individual miRNA changes in isolation. Because circulating miRNAs are highly correlated, global patterns of expression should be studied by representing miRNA data as co-expression networks. MiRNA signatures rather than individual miRNAs may be more reliable biomarkers for cardiovascular events.

**Platelets - an underexplored miRNA-ome.** It is noteworthy that several of the most abundant platelet miRNAs have previously been implicated in cardiovascular pathologies: miR-126 as master regulator of endothelial homeostasis and vascular integrity, miR-21 as mediator of cardiac fibrosis (28) -although this is contested by others (21)- and miR-24 as inducer of endothelial apoptosis after myocardial infarction (7). The presence of these miRNAs within platelets, however, was not taken into account when interpreting these studies. Although the functional importance of platelet miRNAs is currently unknown, it is conceivable that systemic inhibition of miRNAs, which are also abundant in platelets, may alter platelet function and contribute to the observed cardiovascular phenotypes. Also, care must be taken in the design of case control studies for biomarker analysis. Comparisons of circulating miRNAs between patients with manifest cardiovascular disease and healthy controls, for example, are likely to be confounded by medication, in particular anti-platelet therapy.

**Platelet miRNAs as biomarkers.** Combination therapy (aspirin + P2Y₁₂ inhibitors) is commonly used for the treatment of acute coronary syndromes and the prevention of coronary events after placement of a stent, but there is still no generally accepted ideal measure of platelet activation that is widely used as co-diagnostic. Aspirin inhibits the production of thromboxanes. P2Y₁₂ inhibitors such as clopidogrel, prasugrel, ticagrelor act by inhibiting adenosine receptors. Thus, their mechanisms are complementary and the reiterative platelet inhibition increases the likelihood of bleeding, which becomes life threatening in some patients. In contrast, subjects with genetic variants of the liver enzyme CYP2C19 are not protected because clopidogrel is not efficiently converted into an active metabolite (Mega JL et al., N Engl J Med. 2009, 360:354-362; Mega JL et al., Jama. 2012, 307:1482-1483). Following platelet activation, platelet microparticle (PMP) shedding starts within minutes into the circulation (Tans G, et al., Blood. 1991, 77:2641-2648; Heijnen HF et al., Blood. 1999, 94:3791-3799). Because of their small size and instability, PMPs are difficult to measure and not suitable for routine clinical testing. In contrast, miRNAs are stable and readily determined by qPCR. Platelet miRNAs can therefore be useful as a point-of-care test for tailoring anti-platelet therapies. This would address an unmet clinical need for more reliable biomarkers of platelet activation to reduce the risk of life-threatening bleeding complications whilst ensuring the efficacy of treatment. Additional clinical applications of a platelet miRNA assay include diagnosing platelet-related disorders, monitoring thrombus resolution or recurrence, and exploring patient compliance with prescribed anti-platelet therapy.

### References

1. Ai J, Zhang R, Li Y, Pu J, Lu Y, Jiao J, Li K, Yu B, Li Z, Wang R, Wang L, Li Q, Wang N, Shan H, and Yang B. Circulating microRNA-1 as a potential novel biomarker for acute myocardial infarction. Biochem Biophys Res Commun 391: 73-77, 2010.
2. Bartel DP. MicroRNAs: target recognition and regulatory functions. Cell 136: 215-233,2009.
3. Bonauer A, Boon RA, and Dimmeler S. Vascular microRNAs. Curr Drug Targets 11: 943-949, 2010.
4. Care A, Catalucci D, Felicetti F, Bonci D, Addario A, Gallo P, Bang ML, Segnalini P, Gu Y, Dalton ND, Elia L, Latronico MV, Hoydal M, Autore C, Russo MA, Dorn GW, 2nd, Ellingsen O, Ruiz-Lozano P, Peterson KL, Croce CM, Peschle C, and Condorelli G. MicroRNA-133 controls cardiac hypertrophy. Nat Med 13: 613-618, 2007.
5. D'Alessandra Y, Devanna P, Limana F, Straino S, Di Carlo A, Brambilla PG, Rubino M, Carena MC, Spazzafumo L, De Simone M, Micheli B, Biglioli P, Achilli F, Martelli F, Maggiolini S, Marenzi G, Pompilio G, and Capogrossi MC. Circulating microRNAs are new and sensitive biomarkers of myocardial infarction. Eur Heart J 31: 2765-2773, 2010.
6. Fichtlscherer S, De Rosa S, Fox H, Schwietz T, Fischer A, Liebetrau C, Weber M, Hamm CW, Roxe T, Muller-Ardogan M, Bonauer A, Zeiher AM, and Dimmeler S. Circulating microRNAs in patients with coronary artery disease. Circ Res 107:677-684,2010.
7. Fiedler J, Jazbutyte V, Kirchmaier BC, Gupta SK, Lorenzen J, Hartmann D, Galuppo P, Kneitz S, Pena JT, Sohn-Lee C, Loyer X, Soutschek J, Brand T, Tuschl T, Heineke J, Martin U, Schulte-Merker S, Ertl G, Engelhardt S, Bauersachs J, and Thum T. MicroRNA-24 Regulates Vascularity After Myocardial Infarction. Circulation 2011.
8. Fish JE, Santoro MM, Morton SU, Yu S, Yeh RF, Wythe JD, Ivey KN, Bruneau BG, Stainier DY, and Srivastava D. miR-126 regulates angiogenic signaling and vascular integrity. Dev Cell 15: 272-284, 2008.
9. Friedewald WT, Levy RI, and Fredrickson DS. Estimation of the concentration of low-density lipoprotein cholesterol in plasma, without use of the preparative ultracentrifuge. Clin Chem 18: 499-502, 1972.
10. Goeman JJ. L1 penalized estimation in the Cox proportional hazards model. Biom J 52: 70-84, 2010.
11. IHD Register: Report of the Fifth Working Group. Copenhagen DWHO ROfE. 1971.
12. Inui M, Martello G, and Piccolo S. MicroRNA control of signal transduction. Nat Rev Mol Cell Biol 11: 252-263, 2010.
13. Johnnidis JB, Harris MH, Wheeler RT, Stehling-Sun S, Lam MH, Kirak O, Brummelkamp TR, Fleming MD, and Camargo FD. Regulation of progenitor cell proliferation and granulocyte function by microRNA-223. Nature 451: 1125-1129, 2008.
14. Kiddle SJ, Windram OP, McHattie S, Mead A, Beynon J, Buchanan-Wollaston V, Denby KJ, and Mukherjee S. Temporal clustering by affinity propagation reveals transcriptional modules in Arabidopsis thaliana. Bioinformatics 26: 355-362,2010.
15. Kiechl S, Lorenz E, Reindl M, Wiedermann CJ, Oberhollenzer F, Bonora E, Willeit J, and Schwartz DA. Toll-like receptor 4 polymorphisms and atherogenesis. N Engl J Med 347: 185-192, 2002.
16. Latronico MV, Catalucci D, and Condorelli G. Emerging role of microRNAs in cardiovascular biology. Circ Res 101: 1225-1236, 2007.
17. Lehmann U, Streichert T, Otto B, Albat C, Hasemeier B, Christgen H, Schipper E, Hille U, Kreipe HH, and Langer F. Identification of differentially expressed microRNAs in human male breast cancer. BMC Cancer 10: 109, 2010.
18. Lim LP, Lau NC, Garrett-Engele P, Grimson A, Schelter JM, Castle J, Bartel DP, Linsley PS, and Johnson JM. Microarray analysis shows that some microRNAs downregulate large numbers of target mRNAs. Nature 433: 769-773, 2005.
19. Liu N, and Olson EN. MicroRNA regulatory networks in cardiovascular development. Dev Cell 18: 510-525, 2010.
20. Mitchell PS, Parkin RK, Kroh EM, Fritz BR, Wyman SK, Pogosova-Agadjanyan EL, Peterson A, Noteboom J, O'Briant KC, Allen A, Lin DW, Urban N, Drescher CW, Knudsen BS, Stirewalt DL, Gentleman R, Vessella RL, Nelson PS, Martin DB, and Tewari M. Circulating microRNAs as stable blood-based markers for cancer detection. Proc Natl Acad Sci U S A 105: 10513-10518, 2008.
21. Patrick DM, Montgomery RL, Qi X, Obad S, Kauppinen S, Hill JA, van Rooij E, and Olson EN. Stress-dependent cardiac remodeling occurs in the absence of microRNA-21 in mice. J Clin Invest 120: 3912-3916, 2010.
22. Pillai RS, Bhattacharyya SN, and Filipowicz W. Repression of protein synthesis by miRNAs: how many mechanisms? Trends Cell Biol 17: 118-126, 2007.
23. Prokopi M, Pula G, Mayr U, Devue C, Gallagher J, Xiao Q, Boulanger CM, Westwood N, Urbich C, Willeit J, Steiner M, Breuss J, Xu Q, Kiechl S, and Mayr M. Proteomic analysis reveals presence of platelet microparticles in endothelial progenitor cell cultures. Blood 114: 723-732, 2009.
24. Pulikkan JA. Cell-cycle regulator E2F1 and microRNA-223 comprise an autoregulatory negative feedback loop in acute myeloid leukemia. Blood 115: 1768-1778, 2010.
25. Rautou PE, Vion AC, Amabile N, Chironi G, Simon A, Tedgui A, and Boulanger CM. Microparticles, vascular function, and atherothrombosis. Circ Res 109: 593-606, 2011.
26. Scapoli L, Palmieri A, Lo Muzio L, Pezzetti F, Rubini C, Girardi A, Farinella F, Mazzotta M, and Carinci F. MicroRNA expression profiling of oral carcinoma identifies new markers of tumor progression. Int J Immunopathol Pharmacol 23: 1229-1234,2010.
27. Sun W, Shen W, Yang S, Hu F, Li H, and Zhu TH. miR-223 and miR-142 attenuate hematopoietic cell proliferation, and miR-223 positively regulates miR-142 through LMO2 isoforms and CEBP-beta. Cell Res 20: 1158-1169, 2010.
28. Thum T, Gross C, Fiedler J, Fischer T, Kissler S, Bussen M, Galuppo P, Just S, Rottbauer W, Frantz S, Castoldi M, Soutschek J, Koteliansky V, Rosenwald A, Basson MA, Licht JD, Pena JT, Rouhanifard SH, Muckenthaler MU, Tuschl T, Martin GR, Bauersachs J, and Engelhardt S. MicroRNA-21 contributes to myocardial disease by stimulating MAP kinase signalling in fibroblasts. Nature 456: 980-984, 2008.
29. Tijsen AJ, Creemers EE, Moerland PD, de Windt LJ, van der Wal AC, Kok WE, and Pinto YM. MiR423-5p as a circulating biomarker for heart failure. Circ Res 106: 1035-1039, 2010.
30. Wang GK, Zhu JQ, Zhang JT, Li Q, Li Y, He J, Qin YW, and Jing Q. Circulating microRNA: a novel potential biomarker for early diagnosis of acute myocardial infarction in humans. Eur Heart J 31: 659-666, 2010.
31. Wang K, Zhang S, Marzolf B, Troisch P, Brightman A, Hu Z, Hood LE, and Galas DJ. Circulating microRNAs, potential biomarkers for drug-induced liver injury. Proc Natl Acad Sci U S A 106: 4402-4407, 2009.
32. Zampetaki A, Kiechl S, Drozdov I, Willeit P, Mayr U, Prokopi M, Mayr A, Weger S, Oberhollenzer F, Bonora E, Shah A, Willeit J, and Mayr M. Plasma microRNA profiling reveals loss of endothelial miR-126 and other microRNAs in type 2 diabetes. Circ Res 107: 810-817, 2010.
33. Kloosterman WP, Plasterk RH. Dev Cell. 2006 Oct;11(4):441-50.
34. Stefani G, Slack FJ. Nat Rev Mol Cell Biol. 2008 Mar;9(3):219-30.
35. Latronico MV, Catalucci D, Condorelli G. Circ Res. 2007 Dec 7;101(12):1225-36.
36. van Rooij E, Marshall WS, Olson EN. Circ Res. 2008 Oct 24;103(9):919-28.
37. Kuehbacher A, Urbich C, Zeiher AM, Dimmeler S. Circ Res. 2007 Jul 6;101(1):59-68.
38. Tanaka M et al. PLoS One. 2009;4(5):e5532.
39. Laterza OF et al. Clin Chem. 2009 Nov;55(11):1977-83.
40. Ji X, Takahashi R, Hiura Y, Hirokawa G, Fukushima Y, Iwai N. Clin Chem. 2009 Nov;55(11):1944-9.
41. Hoak JC. Platelet and atherosclerosis. Semin. Thromb. Hemost. 1988; 14: 202-5.
42. Rabbani LE, Loscalzo J. Atherosclerosis 1994; 105: 1-7.
43. White JG. Platelets and atherosclerosis. Eur. J. Clin. Invest. 1994; 24 (Suppl 1): 25-9.
44. Falk E, Fernandez-Ortiz A. Role of thrombosis in atherosclerosis and its complications. Am. J. Cardiol. 1995; 75:3B-11B.
45. Nash GF, Turner LF, Scully MF, Kakkar AK: Platelets and cancer. Lancet Oncol 2002, 3(7):425-430.
46. Sierko E, Wojtukiewicz MZ: Platelets and angiogenesis in malignancy. Semin. Thromb. Hemost. 2004, 30(1):95-108
47. Jurasz P, North S, Venner P, Radomski MW: Matrix metalloproteinase-2 contributes to increased platelet reactivity in patients with metastatic prostate cancer: a preliminary study. Thromb. Res. 2003;112(1-2):59-64.

**Table 1. Baseline characteristics of the Bruneck Study population.**

| **Variables** | **All participants** | **Incident myocardial infarction** | | **P value** |
|---|---|---|---|---|
| | (n=820) | Yes (n=47) | No (n=773) | |
| **Demographics and life-style** | | | | |
| Age, years | 62.9(11.1) | 70.0 (9.6) | 62.4(11.1) | <0.001 |
| Female sex, n (%) | 409 (49.9) | 17(36.2) | 392 (50.7) | 0.070 |
| Current/ex-smoker, n (%) | 372 (45.4) | 23 (48.9) | 349 (45.2) | 0.652 |
| Alcohol consumption, g/d | 23.8 (31.2) | 25.0(34.1) | 23.7 (31.0) | 0.781 |

| **Physical examination** | | | | |
|---|---|---|---|---|
| Body mass index, kg/m² | 25.7(3.9) | 26.4 (4.9) | 25.6 (3.8) | 0.165 |
| Waist-hip ratio, cm/cm | 0.9(0.1) | 1.0 (0.1) | 0.9 (0.1) | 0.012 |
| Systolic blood pressure, mmHg | 148.2 (20.6) | 155.9 (22.9) | 147.7 (20.4) | 0.008 |
| Diastolic blood pressure, | 87.1 (9.1) | 87.7 (8.9) | 87.0 (9.2) | 0.646 |
| mmHg | | | | |

| **Lipid markers** | | | | |
|---|---|---|---|---|
| Total cholesterol, mmol/L | 6.0 (1.1) | 6.4 (1.4) | 5.9 (1.1) | 0.003 |
| HDL cholesterol, mmol/L | 1.5 (0.4) | 1.5 (0.5) | 1.5 (0.4) | 0.762 |
| LDL cholesterol, mmol/L | 3.8(1.0) | 4.1 (1.3) | 3.7 (1.0) | 0.033 |
| Triglycerides, mmol/L* | 1.3 ( 1.7) | 1.4 (1.7) | 1.3 (1.7) | 0.223 |
| Apolipoprotein A-I, g/L | 1.7 (0.3) | 1.7 (0.3) | 1.7 (0.3) | 0.926 |
| Apolipoprotein B, g/L | 1.2 (0.3) | 1.3 (0.4) | 1.2 (0.3) | 0.004 |

| **Inflammatory markers** | | | | |
|---|---|---|---|---|
| Leukocytes, x 10⁹/L | 6.5 (1.7) | 6.9 (1.5) | 6.5 (1.7) | 0.111 |
| C-reactive protein, nmol/L* | 17.1 (2.8) | 22.2 (2.5) | 16.8 (2.8) | 0.070 |
| Fibrinogen, g/L | 2.9 (0.8) | 3.1 (0.6) | 2.9 (0.8) | 0.023 |

| **Diabetes** | | | | |
|---|---|---|---|---|
| Type 2 Diabetes, n (%) | 82 (10.0) | 6(12.8) | 76 (9.8) | 0.457 |
| HbAlc, % | 5.8(3.7) | 6.7 (7.5) | 5.7(3.4) | 0.074 |

| **Previous diseases** | | | | |
|---|---|---|---|---|
| Cardiovascular diseases, n (%) | 57 (7.0) | 12 (25.5) | 45 (5.8) | <0.001 |

| | | | | |
|---|---|---|---|---|
| Values are means (SD) or numbers (percentages) unless indicated otherwise. * Variables were logₑ-transformed for analysis and are presented as geometric mean (geometric SD). | | | | |

**Table 2A. Interaction analysis of the association between selective miRNAs and myocardial infarction (MI).**

| **Subgroups** | **Hazard ratio (95% CI) for myocardial infarction per 1 SD increase of logₑ miRNA*** | | |
|---|---|---|---|
| | **MiR-126** | **MiR-197** | **MiR-223** |
| **Diagnosis of diabetes** | | | |
| Yes | 0.23 (0.09-0.61) | 1.06 (0.36-3.14) | 1.46 (0.61-3.51) |
| No | 0.43 (0.23-0.79) | 1.86 (1.04-3.34) | 2.31 (1.31-4.07) |
| | P_{interaction}=0.131 | P_{interaction}=0.268 | P_{interaction}=0.286 |
| | | | |

| **Sex** | | | |
|---|---|---|---|
| Male | 0.39 (0.21-0.73) | 1.57 (0.86-2.89) | 1.95 (1.05-3.63) |
| Female | 0.42 (0.20-0.90) | 2.50 (1.14-5.51) | 2.68 (1.30-5.53) |
| | P_{interaction}= 0.814 | P_{interaction}=0.209 | P_{interaction}=0- 392 |
| | | | |

| **History of cardiovascular disease** | | | |
|---|---|---|---|
| Yes | 0.41 (0.22-0.76) | 2.02 (1.10-3.70) | 2.29 (1.28-4.08) |
| No | 0.30 (0.12-0.79) | 1.19 (0.53-2.70) | 1.83 (0.74-4.54) |
| | P_{interaction}=0.486 | P_{interaction}=0.189 | P_{interaction}=0.624 |

| | | | |
|---|---|---|---|
| * All Cox models included an interaction term of diabetes, sex or history of cardiovascular disease with the miRNA of interest, plus the variables age, sex, smoking status (ever vs. never smokers), systolic blood pressure, LDL cholesterol, diabetes, history of cardiovascular disease, miR-126, miR-197 and miR-223. | | | |

**Table 2B. Interaction analysis of the association between selected miRNAs and incident myocardial infarction (MI).**

| **Subgroups** | | | **Hazard ratio (95 % CI) for myocardial infarction per 1 SD increase of logₑ miRNA*** | | |
|---|---|---|---|---|---|
| | **No. of MI events** | **Incidence rate (per 1000 person-years)** | **MiR-126** | **MiR-197** | **MiR-223** |
| **Diagnosis of diabetes** | | | | | |
| Yes | 6 | 7.9 (3.6-17.6) | 4.07(1.63-10.13) | 0.85 (0.32-2.25) | 0.70(0.26-1.85) |
| No | 41 | 5.2 (3.8-7.0) | 2.35 (1.28-4.30) | 0.55 (0.32-0.94) | 0.47(0.29-0.75) |
| | | | P_{interaction}=0.174 | P_{interaction}=0.337 | P_{interaction}=0.405 |

| **Sex** | | | | | |
|---|---|---|---|---|---|
| Male | 30 | 7.2 (5.0-10.3) | 2.61 (1.40-4.88) | 0.64 (0.36-1.12) | 0.52(0.31-0.88) |
| Female | 17 | 3.7 (2.3-6.0) | 2.36 (1.12-4.96) | 0.42(0.21-0.85) | 0.42(0.23-0.77) |
| | | | P_{interaction}=0.755 | P_{interaction}=0.206 | P_{interaction}=0.491 |

| **History of cardiovascular disease** | | | | | |
|---|---|---|---|---|---|
| Yes | 12 | 25.0 (14.2-44 0) | 2.45 (1.34-4 47) | 0.81(0.37-1.80) | 0.47(0.29-0.76) |
| No | 35 | 4.3 (3.1-5.9) | 3.25 (1.28-8.25) | 0.52 (0.30-0.90) | 0.55(0.24-1.23) |
| | | | P_{interaction}=0.490 | P_{interaction}=0.252 | P_{interaction}=0.711 |

| | | | | | |
|---|---|---|---|---|---|
| All Cox models included an interaction term of diabetes, sex or history of cardiovascular disease with the miRNA of interest, plus the variables age, sex, smoking status (ever vs. never smokers), systolic blood pressure, LDL cholesterol, diabetes, history of cardiovascular disease, miR-126, miR-197 and miR-223 (multivariable model 2). | | | | | |

**Table 3. Clinical characteristics of the healthy volunteers (n=11).**

| | |
|---|---|
| **Male, n (%)** | 9 (82%) |
| **Age, years** | 30 ± 12.2 |
| **Sodium, mmol/L** | 141.36 ± 2.06 |
| **Potassium, mmol/L** | 4.65 ± 0.35 |
| **Urea, mmol/L** | 5.58 ± 0.73 |
| **Creatinine, µmol/L** | 76.28 ± 29.43 |
| **Calcium, mmol/L** | 2.23 ± 0.08 |
| **Albumin, g/dL** | 42.83 ± 13.66 |
| **Alkaline phosphatase, IU/L** | 56 ± 21.82 |
| **Bilirubin, mg/dL** | 10.81 ± 9.67 |
| **Alanine transaminase, IU/L** | 25.72 ± 17.77 |
| **Gamma-glutamyl transpeptidase, IU/L** | 24.27 ± 13.61 |
| **Glucose, mg/dL** | 5.12 ± 0.81 |
| INR | 1.02 ± 0.06 |
| **Total Cholesterol, mg/L** | 4.43 ± 1.27 |
| **HDL, mmol/L** | 1.56 ± 0.6 |
| **LDL, mmol/L** | 2.5 ± 0.98 |
| **Trigycerides, mmol/L** | 0.83 ± 0.53 |
| **C-reactive protein, mg/dL** | <0.5 |

| | |
|---|---|
| INR indicates International Normalized Ratio | |

**Table 4. Correlation between D dimer concentration and expression levels of selected miRNAs***

| | **D-Dimer / miR126** | | **D-Dimer / miR197** | | **D-Dimer/ miR223** | | **D-Dimer / miR24** | | **D-Dimer / miR130a** | | **D-Dimer / miR433** | | **D-Dimer/let7d** | |
|---|---|---|---|---|---|---|---|---|---|---|---|---|---|---|
| **Baseline** | 0.8096 | (0.015) | 0.7024 | (0.052) | 0.9078 | (0.002) | 0.8482 | (0.008) | 0.8436 | (0.009) | 0.7612 | (0.028) | 0.7870 | (0.021) |
| **10 min** | 0.7733 | (0.024) | 0.2085 | (0.620) | 0.7552 | (0.030) | 0.6824 | (0.062) | 0.7265 | (0.041) | 0.7047 | (0.051) | 0.6959 | (0.055) |
| **1 h** | -0.2399 | (0.604) | -0.5388 | (0.212) | -0.433 | (0.333) | -0.491 | (0.263) | -0.2685 | (0.560) | 0.1468 | (0.754) | -0.3104 | (0.498) |
| **5 h** | 0.0876 | (0.852) | -0.1272 | (0.786) | 0.0668 | (0.887) | 0.0999 | (0.831) | 0.1120 | (0.811) | 0.0381 | (0.935) | 0.0932 | (0.842) |
| **2 d** | 0.1816 | (0.667) | 0.0298 | (0.944) | 0.0491 | (0.908) | 0.1393 | (0.742) | 0.4683 | (0.242) | 0.4249 | (0.294) | 0.4663 | (0.244) |
| **7 d** | 0.2535 | (0.511) | -0.0237 | (0.952) | 0.1219 | (0.755) | 0.2483 | (0.519) | 0.1791 | (0.645) | 0.2990 | (0.434) | 0.2952 | (0.441) |

| | | | | | | | | | | | | | | |
|---|---|---|---|---|---|---|---|---|---|---|---|---|---|---|
| * Numbers are age-and sex-adjusted partial correlation coefficients (P values). | | | | | | | | | | | | | | |

**Table 5**

| **MiR combinations** | **AIC** | **C index change** | **NRI** | **IDI** |
|---|---|---|---|---|
| MiR-24, -126, -197 | 564.9772949 | 0.0308 (-0.0290, 0.0907) | 16.54 (-2.32, 35.40) | 0.0555 (0.0076, 0.1034) |
| MiR-24, -126, -150 | 566.5112915 | 0.0217 (-0.0424, 0.0857) | 17.24 (-2.05, 36.53) | 0.0515 (0.0089, 0.0941) |
| MiR-126, -197, -223 | 565.0547485 | 0.0366 (-0.0158, 0.0889) | 16.86 (-1.99, 35.71) | 0.0473 (0.0050, 0.0896) |
| MiR-3423p, -24, - 126 | 567.0376587 | 0.0238 (-0.0326, 0.0802) | 19.37 (0.54, 38.19) | 0.0470 (0.0094, 0.0845) |
| MiR-24, -126, -146b | 566.605957 | 0.0188 (-0.0381, 0.0757) | 16.70 (-1.18, 34.58) | 0.0447 (0.0073, 0.0822) |
| MiR-320, -24, -126 | 565.9118652 | 0.0226 (-0.0325, 0.0777) | 17.08 (-1.29, 35.45) | 0.0434 (0.0095, 0.0773) |
| MiR-191, -24, -126 | 567.0245972 | 0.0195 (-0.0357, 0.0747) | 14.26 (-3.13, 31.64) | 0.0430 (0.0075, 0.0785) |
| MiR-283p, -24, -126 | 566.0445557 | 0.0213 (-0.0333, 0.0760) | 13.78 (-3.59, 31.15) | 0.0427 (0.0077, 0.0778) |
| MiR-25, -24, -126 | 567.6636963 | 0.0182 (-0.0381, 0.0746) | 13.14 (-4.25, 30.54) | 0.0419 (0.0050, 0.0788) |
| let7e, miR-24, -126 | 566.3652344 | 0.0212 (-0.0327, 0.0752) | 11.81 (-5.07, 28.69) | 0.0415 (0.0075, 0.0756) |
| MiR-486, -24, -126 | 566.9111938 | 0.0209 (-0.0324, 0.0741) | 11.33 (-5.54, 28.21) | 0.0410 (0.0055, 0.0766) |
| MiR-24, -126, -454 | 567.1853027 | 0.0197 (-0.0354, 0.0749) | 13.62 (-3.77, 31.01) | 0.0407 (0.0058, 0.0755) |
| MiR-140, -24, -126 | 567.2825928 | 0.0202 (-0.0346, 0.0750) | 13.78 (-3.60, 31.16) | 0.0406 (0.0060, 0.0752) |
| MiR-24, -126, -223 | 567.4731445 | 0.0188 (-0.0355, 0.0732) | 16.07 (-1.81, 33.94) | 0.0404 (0.0059, 0.0749) |
| MiR-122, -24, -126 | 567.4348755 | 0.0199 (-0.0350, 0.0748) | 13.46 (-3.92, 30.84) | 0.0402 (0.0055, 0.0749) |
| MiR-93, -24, -126 | 567.1271362 | 0.0198 (-0.0348, 0.0744) | 13.62 (-3.77, 31.01) | 0.0401 (0.0058, 0.0745) |
| let7b, miR-24, -126 | 567.6226196 | 0.0215 (-0.0332, 0.0763) | 13.94 (-3.44, 31.32) | 0.0398 (0.0062, 0.0734) |
| MiR-21, -126, -197 | 565.9361572 | 0.0373 (-0.0160, 0.0905) | 15.37 (-2.03, 32.77) | 0.0394 (-0.0003, 0.0791) |
| MiR-93, -126, -197 | 564.0219116 | 0.0448 (-0.0055, 0.0951) | 16.54 (-1.34, 34.43) | 0.0392 (0.0020, 0.0764) |
| MiR-320, -126, -223 | 567.2560425 | 0.0211 (-0.0220, 0.0642) | 20.32 (2.44, 38.21) | 0.0310 (0.0069, 0.0551) |

## Claims

1. A method of determining platelet activity in an individual comprising determining in a plasma or serum sample obtained from an individual the level of at least one microRNA selected from the group consisting of miR-126, miR-197, miR-223, miR-24 and miR-21.

2. A method of monitoring the efficacy of an anti-platelet therapy, the method comprising the steps of determining the level of at least one microRNA selected from the group consisting of miR-126, miR-197, miR-223, miR-24 and miR-21 in a plasma or serum sample obtained from an individual before the therapy, determining the level of at least one microRNA selected from the group consisting of miR-126, miR-197, miR-223, miR-24 and miR-21 in a plasma or serum sample obtained from an individual during or after the therapy, and comparing the determined levels in the individual before and during or after the therapy.

3. A method of predicting and/or diagnosing a platelet-related disorder comprising determining in a plasma or serum sample obtained from an individual the level of at least one microRNA selected from the group consisting of miR-126, miR-197, miR-223, miR-24 and miR-21, and comparing the determined level in the individual with a control level.

4. A method of monitoring the progression of a platelet-related disorder comprising the steps of determining the level of at least one microRNA selected from the group consisting of miR-126, miR-197, miR-223, miR-24 and miR-21 in a plasma or serum sample obtained from an individual at a first time point, determining the level of at least one microRNA selected from the group consisting of miR-126, miR-197, miR-223, miR-24 and miR-21 in a plasma or serum sample obtained from an individual at a second time point, and comparing the determined levels in the individual at the first and second time points.

5. A method according to any preceding claim, wherein the levels of a plurality of microRNAs selected from the group consisting of miR-126, miR-197, miR-223, miR-24 and miR-21 are determined.

6. A method according to any one of claims 1 to 4, wherein the level of at least two microRNAs selected from the group comprising miR-126, miR-197 and miR-223 or miR-24 are determined.

7. A method according to claim 6, wherein the levels of:
i) miR-126, miR-197 and miR-223;
ii) miR-126, miR-197 and miR-24;
iii) miR-126, miR-24 and miR-150;
iv) miR-223, miR-126 and miR-93; or
v) miR-126, miR-197, miR-223 and miR-24, are determined.

8. A method according to claim 6, wherein the levels of i) miR-126, miR-197 and miR-223; or ii)miR-126, miR-197 and miR-24, are determined.

## Patentansprüche

1. Verfahren zum Bestimmen von Thrombozytenaktivität in einem Individuum umfassend Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum erhalten wurde.

2. Verfahren zum Überwachen der Wirksamkeit einer Anti-Thrombozyten-Behandlung, das Verfahren umfassend die Schritte Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum vor der Behandlung erhalten wurde, Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum während oder nach der Behandlung erhalten wurde, und Vergleichen der in dem Individuum bestimmten Level vor und während oder nach der Behandlung.

3. Verfahren zum Vorhersagen und/oder Diagnostizieren einer thrombozytenbezogenen Störung umfassend Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum erhalten wurde, und Vergleichen des in dem Individuum bestimmten Levels mit einem Kontrolllevel.

4. Verfahren zum Überwachen der Progression einer thrombozytenbezogenen Störung umfassend die Schritte Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum zu einem ersten Zeitpunkt erhalten wurde, Bestimmen des Levels mindestens einer microRNA, die aus der Gruppe bestehend aus miR-126, miR-197, miR-223, miR-24 und miR-21 ausgewählt ist, in einer Plasma- oder Serumprobe, die von einem Individuum zu einem zweiten Zeitpunkt erhalten wurde, und Vergleichen der in dem Individuum bestimmten Level zu dem ersten und dem zweiten Zeitpunkt.

5. Verfahren gemäß einem vorhergehenden Anspruch, wobei die Level einer Vielzahl von microRNAs, die aus der Gruppe bestehend aus miR-126, miR-197, miR-223, miR-24 und miR-21 ausgewählt sind, bestimmt werden.

6. Verfahren gemäß einem der Ansprüche 1 bis 4, wobei die Level von mindestens zwei microRNAs, die aus der Gruppe bestehend aus miR-126, miR-197 und miR-223 oder miR-24 ausgewählt sind, bestimmt werden.

7. Verfahren gemäß Anspruch 6, wobei die Level von:
i) miR-126, miR-197 und miR-223;
ii) miR-126, miR-197 und miR-24;
iii) miR-126, miR-24 und miR-150;
iv) miR-223, miR-126 und miR-93; oder
v) miR-126, miR-197, miR-223 und miR-24, bestimmt werden.

8. Verfahren gemäß Anspruch 6, wobei die Level von i) miR-126, miR-197 und miR-223; oder ii) miR-126, miR-197 und miR-24, bestimmt werden.

## Revendications

1. Procédé de détermination de l'activité des plaquettes chez un individu, comprenant la détermination dans un échantillon de plasma ou de sérum prélevé sur un individu du taux d'au moins un microARN choisi dans le groupe constitué de miR-126, miR-197, miR-223, miR-24 et miR-21.

2. Procédé de surveillance de l'efficacité d'un traitement antiplaquettaire, le procédé comprenant les étapes de détermination du taux d'au moins un microARN choisi dans le groupe constitué de miR-126, miR-197, miR-223, miR-24 et miR-21 dans un échantillon de plasma ou de sérum prélevé sur un individu avant le traitement, de détermination du taux d'au moins un microARN choisi dans le groupe constitué de miR-126, miR-197, miR-223, miR-24 et miR-21 dans un échantillon de plasma ou de sérum prélevé sur un individu pendant ou après le traitement et de comparaison des taux déterminés chez l'individu avant et pendant ou après le traitement.

3. Procédé de prédiction et/ou de diagnostic d'un trouble associé aux plaquettes, comprenant la détermination dans un échantillon de plasma ou de sérum prélevé sur un individu du taux d'au moins un microARN choisi dans le groupe constitué de miR-126, miR-197, miR-223, miR-24 et miR-21, et la comparaison du taux déterminé chez l'individu à un taux de référence.

4. Procédé de surveillance de l'évolution d'un trouble associé aux plaquettes, comprenant les étapes de détermination du taux d'au moins un microARN choisi dans le groupe constitué de miR-126, miR-197, miR-223, miR-24 et miR-21 dans un échantillon de plasma ou de sérum prélevé sur un individu à un premier moment temporel, de détermination du taux d'au moins un microARN choisi dans le groupe constitué de miR-126, miR-197, miR-223, miR-24 et miR-21 dans un échantillon de plasma ou de sérum prélevé sur un individu à un second moment temporel et de comparaison des taux déterminés chez l'individu au premier et au second moment temporel.

5. Procédé selon l'une quelconque des revendications précédentes, dans lequel les taux d'une pluralité de microARN choisis dans le groupe constitué de miR-126, miR-197, miR-223, miR-24 et miR-21 sont déterminés.

6. Procédé selon l'une quelconque des revendications 1 à 4, dans lequel le taux d'au moins deux microARN choisis dans le groupe constitué de miR-126, miR-197 et miR-223 ou miR-24 sont déterminés.

7. Procédé selon la revendication 6, dans lequel les taux de :
i) miR-126, miR-197 et miR-223 ;
ii) miR-126, miR-197 et miR-24 ;
iii) miR-126, miR-24 et miR-150 ;
iv) miR-223, miR-126 et miR-93 ; ou
v) miR-126, miR-197, miR-223 et miR-24, sont déterminés.

8. Procédé selon la revendication 6, dans lequel les taux de i) miR-126, miR-197 et miR-223 ; ou de ii) miR-126, miR-197 et miR-24 sont déterminés.
